# FASCICULE DE BREVET EUROPEEN

(11) **EP 3 225 083 B1**
(45) Date de publication et mention de la délivrance du brevet: **24.03.2021**
(21) Numéro de dépôt: 15801797.0
(22) Date de dépôt: 26.11.2015
(51) Int. Cl.: H05H 1/46

(54) **PROCÉDÉ ET DISPOSITIF DE GÉNÉRATION D'UNE PLURALITÉ DE JETS DE PLASMA FROID À PRESSION ATMOSPHERIQUE**
VERFAHREN UND VORRICHTUNG ZUR ERZEUGUNG MEHRERER KALTPLASMASTRAHLEN BEI ATMOSPHÄRENDRUCK
METHOD AND DEVICE FOR GENERATING A PLURALITY OF COLD-PLASMA JETS AT ATMOSPHERIC PRESSURE

(30) Priorité: 26.11.2014 FR 1461511
(43) Date de publication de la demande: 04.10.2017
(73) Titulaire: Centre National de la Recherche Scientifique (C.N.R.S.), 75016 Paris (FR); Université d'Orléans, 45000 Orléans (FR); Inel, 45410 Artenay (FR)
(72) Inventeur: POUVESLE, Jean-Michel, 45750 Saint-Pryvé-Saint-Mesmin (FR); ROBERT, Eric, 45100 Orléans (FR); DOZIAS, Sébastien, 45140 Saint-Jean-de-la-Ruelle (FR); HUGNOT, Michel, 45000 Orléans (FR); SARRON, Vanessa, 45300 Pithiviers (FR); DARNY, Thibault, 60860 Blicourt (FR)
(74) Mandataire: Marks & Clerk France
(86) Numéro de dépôt international: PCT/EP2015/077834
(87) Numéro de publication internationale: WO 2016/083539

(56) Documents cités:
- WO-A1-00/79843
- US-A1- 2002 187 066
- US-A1- 2013 272 929
- US-A1- 2014 069 459
- US-B1- 6 406 759
- US-B1- 8 267 884

## Description

### DOMAINE DE L'INVENTION

L'invention concerne un dispositif permettant de générer de manière simultanée ou séquentielle une pluralité de jets de plasma, en particulier des jets de plasma froid à pression atmosphérique.

L'invention trouve notamment application dans les domaines suivants : le domaine biomédical, la stérilisation, la médecine, la cosmétique, le traitement de matériaux, la fonctionnalisation de surfaces, la dépollution, la germination, l'éclairage, la commutation rapide, la modification d'écoulements, la détection ou encore la métrologie.

### ARRIERE-PLAN TECHNOLOGIQUE

Un plasma est une phase de la matière comprenant des particules chargées, des ions et des électrons. Typiquement, il s'agit d'un gaz ionisé, c'est-à-dire un gaz comprenant des électrons libres qui ne sont pas liés à un atome ou à une molécule. Les électrons libres rendent le plasma conducteur de l'électricité.

L'invention concerne plus particulièrement les plasmas froids à pression atmosphérique.

Actuellement, la production de jets de plasma froid à pression atmosphérique fait l'objet de nombreuses recherches compte-tenu de l'importance grandissante de leurs applications dans des domaines nombreux et variés, tels que le biomédical, la médecine, la stérilisation, la décontamination ou encore le traitement des matériaux.

Ces jets de plasma froid présentent cependant l'inconvénient de ne permettre de traiter que de petites surfaces, en raison de la petite dimension des jets pouvant être actuellement produits.

On a donc proposé de mettre en œuvre des systèmes de balayage afin de balayer les surfaces à traiter à l'aide du jet de plasma froid produit selon les techniques conventionnelles. Le balayage nécessite toutefois la mise en œuvre d'un système capable de mettre le jet de plasma en mouvement au-dessus de la surface à traiter, ce qui rend le dispositif relativement complexe et augmente considérablement son encombrement.

Il a également été proposé de coupler un grand nombre de jets de plasma à partir de systèmes multi-électrodes et multi-générateurs. Ainsi, afin d'obtenir *n* jets de plasma, cette solution propose d'associer *n* électrodes à autant de générateurs. Un tel couplage permet effectivement d'augmenter la surface de la zone à traiter en multipliant les sources de jets de plasma. Toutefois, afin que les jets de plasma n'interfèrent pas, il est nécessaire d'espacer les électrodes de décharge. Par ailleurs, cette solution est relativement chère, encombrante et complexe à mettre en œuvre en raison de la multiplication des électrodes de décharge et/ou des capillaires et tuyaux d'alimentation en gaz.

La technologie actuelle permet certes de réduire la taille des sources de plasma (voir notamment les dispositifs à microcavités - IEEE TRANSACTIONS ON PLASMA SCIENCE, Vol. 41, n°4, Avril 2013). Toutefois, cette miniaturisation a un coût non négligeable et est très complexe à mettre en œuvre. Elle ne permet en outre pas de générer des plasmas de grandes dimension, ou au contraire suffisamment petits pour traiter des cibles présentant au moins une très petite dimension et un grand rapport d'aspect, tels que des systèmes endoscopique

Le document US 8,267,884 D1 divulgue un exemple de procédé et de système de génération d'une pluralité de jets plasma.

### RESUME DE L'INVENTION

Un objectif de l'invention est donc de proposer un nouveau dispositif et un procédé associé permettant de générer un plasma froid à pression atmosphérique en réduisant le nombre de sources nécessaires pour générer le plasma, qui soit en outre capable de produire des plasmas susceptibles de traiter tout type de cible, indépendamment de leurs dimensions et rapports d'aspect, ainsi que ces cibles comprennent un matériau diélectrique ou conducteur.

Pour cela, l'invention propose un procédé de génération d'une pluralité de jets de plasma froid à pression atmosphérique afin de traiter une cible, selon la revendication 1.

Certaines caractéristiques préférées mais non limitatives du procédé de génération décrit ci-dessus sont divulguées aux revendications dépendantes.

Selon un deuxième aspect, l'invention propose également un dispositif de génération d'une pluralité de jets de plasma froid à pression atmosphérique afin de traiter une cible, selon la revendication 5.

Certaines caractéristiques préférées mais non limitatives du dispositif décrit ci-dessus sont divulguées aux revendications dépendantes.

### BREVE DESCRIPTION DES DESSINS

D'autres caractéristiques, buts et avantages de la présente invention apparaîtront mieux à la lecture de la description détaillée qui va suivre, et au regard des dessins annexés donnés à titre d'exemples non limitatifs et sur lesquels :

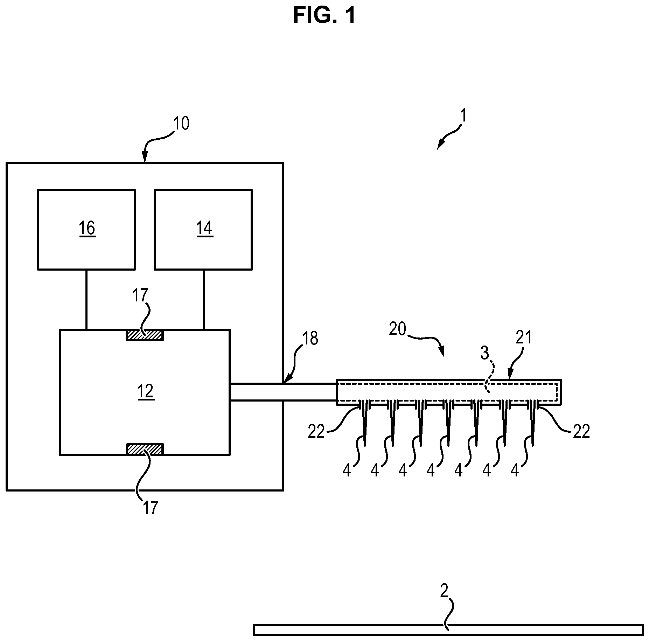

La figure 1 est un schéma d'un exemple de réalisation d'un dispositif de génération d'une pluralité de jets de plasma froid à pression atmosphérique conforme à l'invention.
La figure 2 illustre un premier exemple de réalisation d'un substrat pouvant être utilisé dans le dispositif de la figure 1.
Les figures 3a à 3h illustrent d'autres exemples de réalisation d'un substrat pouvant être utilisé dans le dispositif de la figure 1.

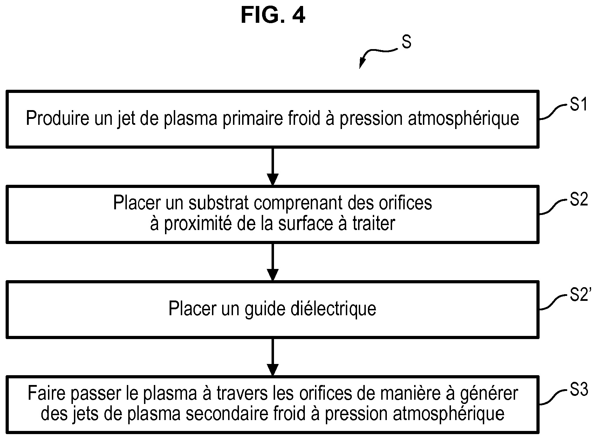

La figure 4 est un organigramme représentant différentes étapes d'un procédé de génération d'une pluralité de jets de plasma froid à pression atmosphérique conforme à l'invention.

### DESCRIPTION DETAILLEE D'UN MODE DE REALISATION

Un procédé S et un dispositif 1 de génération d'une pluralité de jets de plasma froid à pression atmosphérique afin de traiter une cible 2 conformes à l'invention vont à présent être décrits en référence aux figures annexées. La cible 2 peut notamment comprendre une matière physique, un tissu vivant ou un volume de fluide, et peut le cas échéant être posée sur un support mis à la masse afin de modifier la qualité du plasma et faire varier les espèces réactives et le champ électrique qui ont un impact sur la cible 2.

Par plasma froid à pression atmosphérique, on comprendra dans ce qui suit un plasma gazeux hors équilibre thermodynamique pour lequel la température des électrons est très élevée par rapport à la température des autres espèces contenues dans le plasma, cette dernière température restant proche de la température ambiante.

Ainsi, l'invention n'est pas limitée à son application à température ambiante ou à pression ambiante. En particulier, l'invention s'applique mutatis mutandis à la génération de jets de plasma froid à une pression allant d'une centaine de torrs (10 kPa) à plusieurs atmosphères (*i.e.* plusieurs centaines de kPa) et/ou à basse température (soit de préférence inférieure à une centaine de degrés Celsius).

On notera toutefois que la température du procédé S de l'invention peut être limitée par le type de cible 2 à traiter : typiquement, pour des matières biologiques, la température est de préférence inférieure à une quarantaine de degrés Celsius.

Au cours d'une première étape S1, le procédé S comprend la production d'un jet de plasma froid à pression atmosphérique à l'aide d'une source de plasma 10.

Dans ce qui suit, le jet de plasma obtenu par la source de plasma 10 sera désigné par la terminologie de « jet de plasma primaire 3 ».

La source de plasma 10 froid peut notamment être conforme au dispositif de génération de plasma décrit dans le document WO 2009/050240. Toutefois, tout dispositif 1 susceptible de délivrer un jet de plasma froid à pression atmosphérique peut être utilisé comme source de plasma 10.

Ainsi, la source de plasma 10 peut par exemple comprendre une enceinte 12, connectée à une source 14 de gaz plasmagène, dans laquelle sont logées au moins une électrode, par exemple deux électrodes 17 reliées à un générateur 16 à haute tension.

La source 14 de gaz plasmagène peut notamment comprendre une source 14 de gaz rare, d'un mélange de gaz rares (typiquement de l'hélium He, de l'argon, Ar, etc.) ou d'un mélange d'un ou plusieurs gaz rares avec un ou plusieurs gaz moléculaires (typiquement de l'oxygène O2, de l'hydrogène H2, de l'hexafluorure de soufre SF6, de l'azote N2 et de la vapeur d'eau H2O etc.) minoritaires, c'est-à-dire ajoutés à faible concentration.

Le générateur 16 est de préférence configuré pour appliquer une décharge électrique ayant un pic de tension très rapide (typiquement inférieur à quelques microsecondes) entre zéro et plusieurs dizaines de kilovolts, afin de créer un front d'ionisation très rapide au sein du gaz plasmagène. Par ailleurs, le générateur 16 peut appliquer un courant alternatif à des fréquences variées allant d'une cinquantaine de hertz à plusieurs kilohertz. En variante, le générateur 16 peut appliquer un courant impulsionnel et générer des décharges uniques ou répétées à très haute fréquence (allant jusqu'à une centaine de kilohertz). Dans cette variante de réalisation, les électrodes 17 peuvent aussi bien être alimentées par une polarité positive ou négative.

De la sorte, l'application dans l'enceinte 12 d'une décharge électrique par les électrodes 17 dans le flux de gaz plasmagène a pour effet de produire un jet de plasma primaire 3.

Le jet de plasma primaire 3 est ensuite dirigé vers une sortie de l'enceinte 12.

De manière optionnelle, un guide 18 réalisé dans un matériau diélectrique peut être placé au niveau de la sortie de l'enceinte 12, afin d'amener le jet de plasma primaire 3 dans une région située à distance de la source de plasma 10. Dans cette forme de réalisation, le guide 18 contient un gaz ou un mélange de gaz permettant le transport du jet de plasma primaire jusqu'à la région voulue, qui peut être placée à une distance allant de quelques millimètres à plusieurs centimètres ou plusieurs mètres de la source de plasma 10, selon les besoins. Le guide 18 peut notamment avoir la forme d'une enveloppe cylindrique ou tubulaire. Le gaz ou le mélange de gaz contenu dans le guide 18 peut être choisi parmi les gaz plasmagènes susceptible d'être créés par la source 14. Ce gaz contenu dans le guide 18 peut être différent de celui créé par la source 14 et constitue un apport supplémentaire.

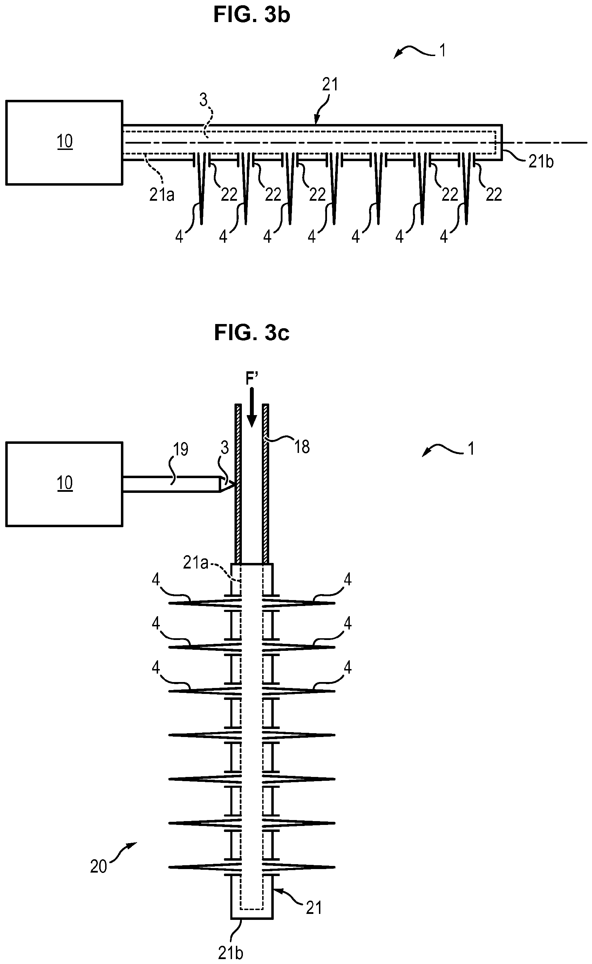

Dans une variante de réalisation illustrée sur la figure 3c, la source de plasma 10 peut être mise en communication fluidique avec le guide diélectrique 18 via un guide intermédiaire 19. Le guide intermédiaire 19 peut alors être diélectrique, conducteur, ou diélectrique et comprendre un insert conducteur.

Pour cela, la source de plasma 10 peut injecter un jet de plasma froid à pression atmosphérique dans le guide intermédiaire 19, qui forme alors au niveau de son extrémité libre un jet de plasma primaire 3 qui est transféré dans le guide diélectrique 18. Par ailleurs, une source de flux de gaz plasmagène F' est mise en communication fluidique avec le guide diélectrique 18 pour injecter le flux F' dans une première extrémité du guide diélectrique 18, tandis que la deuxième extrémité du guide diélectrique 18 est connectée au substrat 20. De la sorte, le jet de plasma primaire 3 issu du guide intermédiaire 19 est transporté par le guide diélectrique 18 jusqu'au substrat 20, où sont produits les différents jets de plasma 4.

De manière alternative, lorsque le guide intermédiaire 19 est réalisé dans un matériau conducteur, il peut être connecté directement à l'une des électrodes 17. Le jet de plasma primaire 3 est alors formé à l'extrémité du guide intermédiaire conducteur 19, puis transféré dans le guide diélectrique 18 dans lequel circule le flux de gaz plasmagène F' issu de la source 14, afin de produire les jets de plasma 4.

Au cours d'une deuxième étape S2, on place un substrat 20 à proximité de la cible 2 à traiter.

Le substrat 20 est configuré pour former, à partir du jet de plasma primaire 3 produit par la source, au moins deux jets de plasma 4, de préférence une pluralité de jets de plasma 4.

A cet effet, le substrat 20 comprend au moins deux orifices 22 traversants, de préférence autant d'orifices 22 traversants qu'il y a de jets de plasma 4 à produire. Les orifices 22 traversants permettent de mettre en communication fluidique la source de plasma 10 avec la cible 2 à traiter.

Le cas échéant, un guide 18 formé dans un matériau diélectrique peut être placé entre la source de plasma 10 et le substrat 20, de manière à transporter le flux de gaz vers les orifices 22 traversants du substrat 20 (étape S2').

On comprendra bien entendu que la première et la deuxième étape S1, S2 peuvent être inversées, le substrat 20 pouvant être placé à proximité de la cible 2 à traiter préalablement à la production du jet de plasma primaire 3 par la source de plasma 10.

Au cours d'une troisième étape S3, on fait alors passer le jet de plasma primaire 3 à travers les orifices 22 traversants de manière à générer au moins deux jets de plasma 4 froid à pression atmosphérique, de préférence une pluralité de jets de plasma 4.

Plus précisément, le jet de plasma primaire 3 est appliqué sur le substrat 20, de manière à passer à travers les différents orifices 22 traversants qui sont formés dans le substrat 20 et générer ainsi les différents jets de plasma 4.

Dans ce qui suit, les jets de plasma 4 formés par passage du jet de plasma primaire 3 dans les orifices 22 traversants du substrat 20 seront désignés par la terminologie « jets de plasma secondaire 4 ».

L'invention permet ainsi, à l'aide d'un seul générateur 16 et d'une unique source 14 de gaz plasmagène, d'obtenir une pluralité de jets de plasma secondaire 4. Le dispositif 1 de l'invention est donc moins complexe et moins cher à réaliser que les systèmes multi-électrodes et multi-générateurs conventionnels. Un seul générateur 16 permet en effet de produire, grâce au substrat 20, plusieurs centaines de jets de plasma secondaire 4.

Le dispositif 1 de l'invention permet en outre de réduire drastiquement la consommation de gaz plasmagène pour la réalisation d'un grand nombre de jets de plasma secondaire 4. Par exemple, un débit entrant de gaz plasmagène de l'ordre d'un litre par minute suffit pour produire une centaine de jets de plasma secondaire 4, tandis que les systèmes multi-électrodes et multi-générateurs de l'art antérieur requièrent un litre par minute de gaz plasmagène par jet de plasma produit (ce qui revient à environ 100 litres par minutes de gaz plasmagène pour la production d'une centaine de jets de plasma produits).

La forme, les dimensions et le matériau constitutif du substrat 20 dépendent du type de cible 2 à traiter.

Le substrat 20 peut notamment être réalisé dans un matériau diélectrique (mode séquentiel), un matériau conducteur (mode simultané), un matériau conducteur recouvert en surface d'un matériau diélectrique, un matériau diélectrique recouvert en surface d'un matériau conducteur, ou encore d'une première partie formée dans un matériau diélectrique et d'une deuxième partie formée dans un matériau conducteur.

La mise en œuvre d'un matériau conducteur de l'électricité, tel qu'un matériau métallique, permet de produire plus des jets de plasma secondaire 4 sensiblement identiques les uns aux autres (c'est-à-dire homogènes), le matériau métallique étant susceptible de conduire les électrons issus du jet de plasma primaire 3. Toutefois, un tel matériau n'est pas adapté à tous les types d'application. En particulier, un substrat 20 en matériau conducteur ne peut être mis en œuvre que si la cible 2 à traiter le permet (au vu des hautes tensions qui sont appliquées par le générateur 16). Ce type de matériau peut par exemple être privilégié pour la réalisation de jets de plasma secondaire 4 à l'échelle industrielle, lorsque la cible 2 à traiter le permet.

La mise en œuvre d'un matériau diélectrique permet au contraire de mettre en œuvre l'invention quelle que soit le type de cible 2 à traiter. Ce type de matériau peut notamment être privilégié pour des applications biologiques et médicales, ou pour des objets considérés comme fragiles. Par exemple, le matériau diélectrique peut comprendre une céramique, du verre, une matière plastique, ou tout matériau diélectrique susceptible d'être usiné et/ou moulé aux formes et aux dimensions désirées pour le substrat 20.

La mise en œuvre d'un substrat 20 formé dans un matériau conducteur recouvert en surface d'un matériau diélectrique présente l'avantage de produire facilement des jets de plasma secondaire 4 homogènes quelle que soit la cible 2 à traiter. Ce type de substrat 20 est cependant plus complexe à réaliser que les substrats 20 exclusivement diélectriques ou conducteurs de l'électricité.

Les cibles 2 pouvant être traitées avec le dispositif 1 et le procédé S de l'invention peuvent donc être de nature très différente, seul le matériau constitutif du substrat 20 devant être adapté (matériau conducteur de l'électricité et/ou diélectrique) au type de cible 2 à traiter. Les cibles 2 pouvant être traitées peuvent donc aussi bien comprendre des matériaux inertes diélectriques ou conducteurs, des tissus biologiques, des organes, etc. Il est même possible de traiter des liquides en surface ou en variante de plonger le substrat 20 directement dans ces liquides.

Par ailleurs, les cibles 2 pouvant être traitées peuvent être de forme et de dimensions variées (de quelques millimètres à quelques mètres), en deux dimensions ou en trois dimensions.

En effet, tout type de cible 2 peut en outre être traité, quel que soit le rapport d'aspect (y compris les cibles 2 comprenant une très petite dimension devant les autres dimensions, telles que les tuyaux et cathéters par exemple) ou les dimensions de la cible 2. Il suffit en effet d'adapter les dimensions et la forme du substrat 20 aux dimensions et à la forme de la cible 2 à traiter.

Le substrat 20 peut en effet présenter des formes diverses et variées suivant le type d'application demandé. En particulier, la forme du substrat 20 et la distribution des orifices 22 peuvent être choisis de manière à obtenir une fontaine plasma, une douche plasma, un rideau plasma, une nappe plasma ou encore un gicleur plasma, de manière simple et peu coûteuse, le substrat 20 pouvant aussi bien avoir une forme bidimensionnelle ou tridimensionnelle.

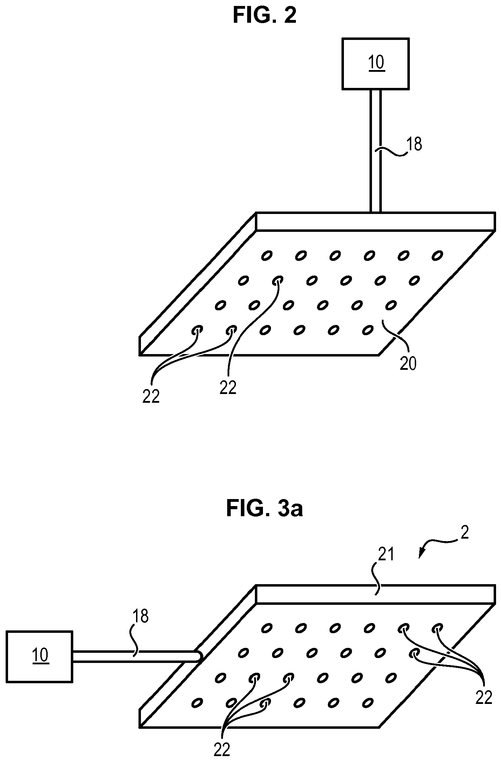

Par exemple, le substrat 20 peut être plan et s'étendre sensiblement perpendiculairement à la direction de propagation du jet de plasma primaire 3 (figure 2).

En variante, le substrat 20 peut comprendre une enveloppe 21 formant un guide présentant une première extrémité 21a configurée pour recevoir le jet de plasma primaire 3 et une deuxième extrémité 21b, qui est opposée à la première extrémité 21a et peut être ouverte ou fermée. Dans cette variante de réalisation, le jet de plasma primaire 3 se déplace entre la première et la deuxième extrémité 21a, 21b de l'enveloppe 21 et produit des jets de plasma secondaire 4 en passant à travers les orifices 22 traversants qui s'étendent entre ces deux extrémités 21a, 21b.

Des exemples d'enveloppes 21 ont été illustrés sur les figures 3a à 3g.

Les figures 3a et 3b illustrent un exemple d'enveloppe 21 de forme cylindrique comprenant une série d'orifices 22 traversants alignés le long de l'enveloppe 21, entre sa première et sa deuxième extrémité 21b, configurés pour former une série de jets de plasma secondaire 4. Ici, la deuxième extrémité 21b de l'enveloppe 21 est fermée.

Dans le cas de la figure 3a, l'enveloppe 21 est de forme sensiblement rectangulaire, et comprend deux faces planes opposées reliées entre elles par au moins trois côtés, entre lesquelles circule le jet de plasma primaire 3. Le jet de plasma primaire 3 est ici introduit par un orifice formé dans l'un des côtés de l'enveloppe 12, les dimensions de l'orifice étant sensiblement plus petites que la longueur des côtés de l'enveloppe 21. Cet exemple de réalisation permet donc d'obtenir une matrice de jets de plasma secondaire 4.

Dans le cas de la figure 3b, l'enveloppe 21 est de forme cylindrique de révolution et les orifices 22 traversants sont alignés le long d'une ligne qui s'étend entre les deux extrémités 21a, 21b de l'enveloppe 21. Cet exemple de réalisation permet donc d'obtenir une ligne de jets de plasma secondaire 4.

La figure 3c illustre un exemple d'enveloppe 21 de forme cylindrique comprenant deux séries d'orifices 22 alignés s'étendant dans un même plan le long de l'enveloppe 21, entre ses deux extrémités 21a, 21b. Cet exemple de réalisation permet donc d'obtenir deux lignes de jets de plasma secondaire 4, se propageant radialement depuis l'enveloppe 21, dans un même plan. Ici encore, la deuxième extrémité 21b de l'enveloppe 21 est fermée.

Cet exemple n'est cependant pas limitatif, les séries d'orifices pouvant s'étendre dans des plans différents.

Dans cet exemple de réalisation, la source de plasma 10 est mise en communication fluidique avec le guide diélectrique 18 via le guide intermédiaire 19. Plus précisément, le jet de plasma primaire 3 est transféré par le guide intermédiaire 19 dans le guide diélectrique 18 puis dans l'enveloppe 21, où sont produits les différents jets de plasma 4. On comprendra bien entendu que cette configuration du dispositif 1 (avec un guide intermédiaire 19) n'est pas limitée au substrat 20 illustré en figure 3c, et peut être appliquée à toute forme de substrat 20.

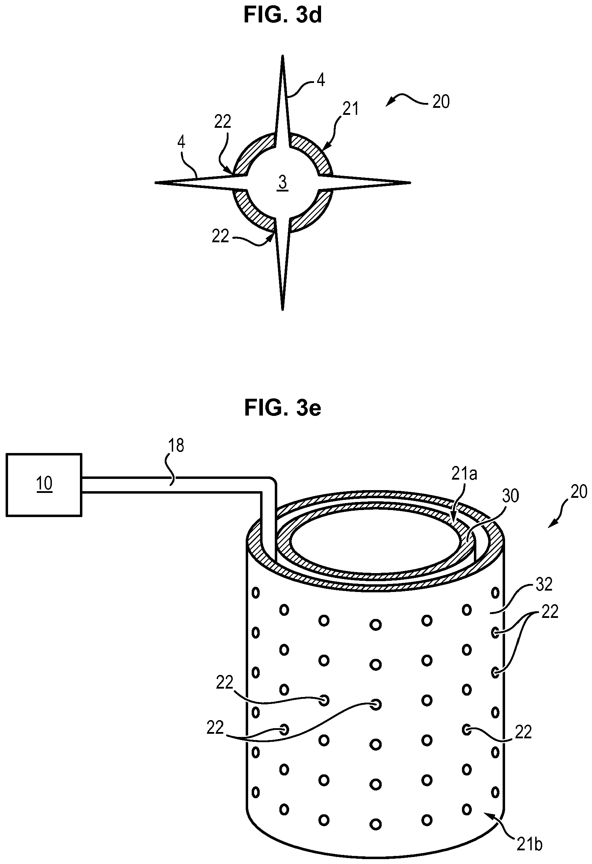

La figure 3d illustre un exemple d'enveloppe 21 comprenant un tube sensiblement cylindrique de révolution dans lequel sont formées plusieurs lignes d'orifices 22 traversants, ici quatre lignes. Cet exemple de réalisation permet donc d'obtenir plusieurs lignes de jets de plasma secondaire 4 s'étendant radialement par rapport à l'enveloppe 21, ici quatre lignes formant une croix.

La figure 3e illustre un exemple d'enveloppe 21, comprenant un tube interne 30 sensiblement continu (c'est-à-dire dépourvu d'orifices 22 traversants) logé dans un tube externe 32 dans lequel sont formés une pluralité d'orifices 22 traversants. Le tube interne 30 et le tube externe 32 sont ici cylindriques de révolution et coaxiaux. Dans cet exemple de réalisation, l'introduction d'un jet de plasma primaire 3 entre le tube interne 30 et le tube externe 32 permet donc d'obtenir une pluralité de jets de plasma secondaire 4 s'étendant radialement depuis le tube externe 32, entre la première et la deuxième extrémité 21a, 21b.

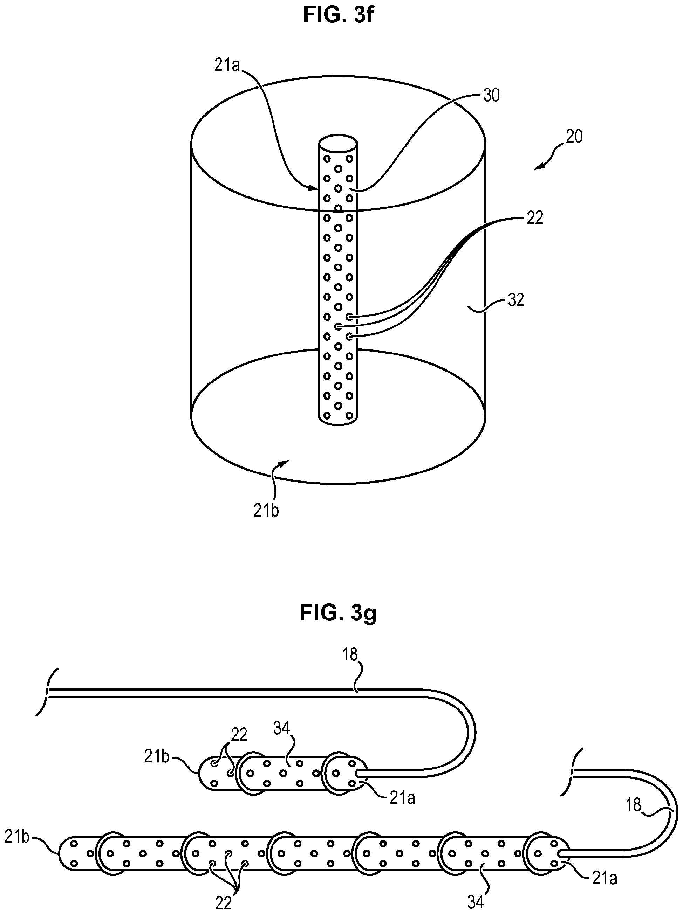

En variante (voir figure 3f), les orifices 22 traversants peuvent être formés dans le tube interne 30 au lieu du tube externe 32 de l'enveloppe 21. Cette variante de réalisation permet notamment de projeter les jets de plasma secondaire 4 ainsi produits dans un milieu confiné afin d'obtenir un plasma diffus.

La figure 3g illustre deux exemples de réalisation dans lesquels l'enveloppe 21 présente la forme d'un goupillon 34 présentant une forme sensiblement cylindrique de révolution et comprenant une pluralité d'orifices 22 traversants répartis sur sa cible 2 entre sa première et sa deuxième extrémité 21b. Le cas échéant, la deuxième extrémité 21b peut être fermée. De manière optionnelle, le goupillon 34 peut être connecté à un guide 18 diélectrique flexible au niveau de sa première extrémité 21a afin de faciliter sa manipulation.

Typiquement, un goupillon 34 peut être mis en œuvre pour le traitement de la surface interne d'une cible 2 comprenant une structure creuse, même si cette structure creuse présente un grand rapport d'aspect, comme c'est le cas par exemple d'un cathéter ou d'un dispositif endoscopique. Il suffit en effet de réaliser un goupillon 34 de dimensions adaptées pour pénétrer dans le cathéter ou le dispositif endoscopique et le traiter.

De manière optionnelle, les goupillons 34 peuvent comprendre des anneaux fixés sur la surface externe de l'enveloppe 21 de manière sensiblement coaxiale avec l'enveloppe 21. Les anneaux permettent d'améliorer la circulation des jets de plasma secondaire lorsque le goupillon est introduit dans la structure creuse de la cible.

Le dispositif 1 et le procédé S de l'invention permettent donc de traiter la cible 2 d'un objet présentant un très grand rapport d'aspect avec une dimension de l'ordre de quelques micromètres, tel qu'un cathéter. En comparaison, les techniques de production conventionnelles de jets de plasma froid ne sont pas capables de traiter de telles cibles 2, dans la mesure où elles ne peuvent pas maitriser suffisamment les dimensions ni la forme des jets ainsi produits pour pouvoir les introduire dans un tel objet.

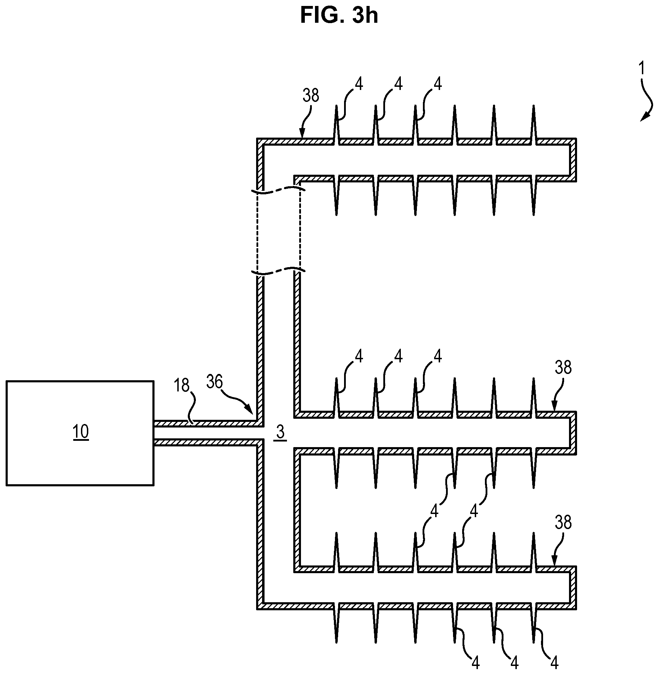

La figure 3h illustre un exemple de réalisation dans lequel le guide diélectrique 18 comprend plusieurs branches 38 connectées ensemble au niveau d'un noyau 36 central. C'est alors le noyau 36 central qui est placé à proximité de la source de plasma 10 et distribue le jet de plasma primaire 3 dans les différentes branches 38. Ici, chaque branche 38 peut comprendre une enveloppe 21 conforme aux exemples de réalisation illustrés en figures 3a à 3g.

Par ailleurs, les orifices 22 traversants d'un même substrat 20 peuvent être de forme et de dimensions différentes. Typiquement, les orifices 22 traversants peuvent être de forme globalement circulaire, ovale, parallélépipédique, etc.

Bien entendu, quelle que soit la forme de réalisation, une enveloppe 21 comprenant un nombre différent de lignes d'orifices 22, ou des orifices 22 distribués suivant une matrice de répartition différente (répartition aléatoires, en quinconce, etc.) de celle décrite en relation avec les figures annexées peut tout aussi bien être mise en œuvre pour la réalisation des jets de plasma secondaire 4.

La position, le diamètre et la profondeur des orifices 22 traversants peuvent par ailleurs être choisis en fonction de l'application souhaitée et/ou du débit du flux de gaz plasmagène entrant dans la source de plasma 10.

Typiquement, le diamètre des orifices 22 peut être compris entre quelques micromètres (par exemple dans le cas de la nano-fonctionnalisation d'une cible 2) et plusieurs millimètres (par exemple dans le cas d'un traitement en dermatologie de tissus biologiques).

Selon les applications, il peut en outre être préférable d'obtenir des jets de plasma secondaire 4 qui n'interfèrent pas les uns avec les autres (c'est-à-dire sans interaction électromagnétique).

Etant donné que la source de plasma 10 produit des jets de plasma secondaire 4 discontinus qui présentent chacun une grande vitesse de propagation (typiquement de l'ordre de quelques 10³ à 10⁶ mètres par secondes (m.s⁻¹)), un choix judicieux de la distance entre deux orifices 22 traversants adjacents, de leur forme et leur profondeur respectives en fonction du débit du flux de gaz plasmagène permet d'éviter des interactions électrostatiques entre les jets de plasma secondaire 4 produits par ces deux orifices 22 traversants adjacents. En effet, plus la distance parcourue par le jet de plasma primaire 3 entre la formation de deux jets de plasma secondaire 4 est grande, moins il y a de risques d'interaction électrostatique. Or, cette distance parcourue peut notamment être ajustée en fonction de la distance entre les orifices 22 traversants adjacents et/ou de la profondeur des orifices 22 traversants (qui dépend de l'épaisseur du substrat 20), selon le débit du flux de gaz plasmagène entrant dans la source de plasma 10.

De la sorte, le jet de plasma secondaire 4 peut être produit par l'orifice 22 traversant le plus proche de la source de plasma 10 avant que le jet de plasma secondaire 4 suivant ne soit formé.

Les Demanderesses se sont aperçues que, en fonction du débit de gaz entrant dans la source de plasma 10, le choix judicieux du rapport d'aspect (longueur sur diamètre) des orifices 22 traversants du substrat 20 influençait le volume de gaz en sortie desdits orifices 22, permettant ainsi de limiter les risques d'interaction entre deux jets de plasma secondaire 4 adjacents. Le rapport d'aspect des orifices 22 peut notamment être compris entre 1 et 100.

Par exemple, les orifices 22 peuvent présenter un rapport d'aspect compris entre 5 et 50 lorsque le débit du flux de gaz entrant dans la source de plasma 10 est compris entre un ou plusieurs centimètres cubes par minutes standards (sccm) et quelques litres par minutes standards (sLm) (dans des conditions de référence de 20°C et 1013 10² Pa).

Les différents jets de plasma secondaire 4 obtenus à l'aide d'un même substrat 20 ne se forment donc pas simultanément mais successivement, en fonction de la vitesse de propagation du jet de plasma primaire 3 produit par la source de plasma 10 et de la position et des dimensions des orifices 22 traversants.

Cette discontinuité dans la formation des jets de plasma secondaire 4 à l'aide du substrat 20 n'a cependant pas d'influence sur le traitement de la cible 2, la durée entre la formation de deux jets de plasma secondaire 4 adjacents étant de l'ordre de quelques nanosecondes à quelques microsecondes. Le traitement de la cible 2 à l'aide des jets de plasma secondaire 4 obtenus suivant le procédé S de l'invention est donc homogène.

Le dispositif 1 et le procédé S de l'invention permettent donc de générer des jets de plasma secondaire 4 de diverses longueurs, allant de quelques dizaines de micromètres à plusieurs centimètres, de manière contrôlée grâce au substrat 20.

## Revendications

1. Procédé (S) de génération d'une pluralité de jets de plasma froid à pression atmosphérique afin de traiter une cible (2), comprenant les étapes suivantes :
- produire (S1) un jet de plasma primaire (3) froid à pression atmosphérique à l'aide d'une source de plasma (10) en générant des décharges électriques dans un flux de gaz plasmagène,
- placer (S2) un substrat (20, 21, 30, 32, 34) à proximité de la cible (2) à traiter, ledit substrat (20, 21, 30, 32, 34) comprenant au moins deux orifices (22) traversants,
- faire passer (S3) le jet de plasma primaire (3) à travers les orifices (22) traversants de manière à générer au moins deux jets de plasma secondaire (4) froid à pression atmosphérique **caractérisé par** un rapport d'aspect des orifices (22) traversants du substrat (20, 21, 30, 32, 34) est-compris entre 1 et 100, de préférence entre 5 et 50, ledit rapport d'aspect étant défini comme un rapport d'une longueur sur un diamètre d'un orifice traversant, en fonction du débit du flux de gaz entrant dans la source de plasma (10) ce dernier étant compris entre un ou plusieurs centimètres cubes par minutes standard (sccm) et quelques litres par minutes standards (slm) dans des conditions de référence de 20°C et 1013 10² Pa.

2. Procédé de génération (S) selon la revendication 1, comprenant en outre une étape au cours de laquelle un guide (18) formé dans un matériau diélectrique est placé entre la source de plasma (10) et le substrat (20, 21, 30, 32, 34), de manière à transporter le flux de gaz vers les orifices (22) traversants dudit substrat (20, 21, 30, 32, 34).

3. Procédé de génération (S) selon la revendication 2, dans lequel le flux de gaz plasmagène comprend un gaz rare, un mélange de gaz rares ou un mélange d'un ou plusieurs gaz rares avec un mélange minoritaire comprenant un ou plusieurs gaz moléculaires.

4. Procédé de génération (S) selon la revendication 3, dans lequel la cible (2) à traiter comprend une structure creuse, le procédé comprenant une sous-étape d'introduction du substrat (34) dans ladite structure creuse préalablement à l'étape (S3) de passage du jet de plasma primaire (3) à travers les orifices (22) traversants.

5. Dispositif (1) de génération d'une pluralité de jets de plasma froid à pression atmosphérique afin de traiter une cible (2), le dispositif comprenant :
- une source de plasma (10), configurée pour produire un jet de plasma primaire (3) froid à pression atmosphérique en générant des décharges électriques dans un flux de gaz plasmagène,
- un substrat (20, 21, 30, 32, 34) comprenant une pluralité d'orifices (22) traversants, et
- des moyens configurés pour faire passer le plasma à travers les orifices (22) traversants du substrat (20, 21, 30, 32, 34) de manière à générer une pluralité de jets de plasma secondaire (4) froid à pression atmosphérique,
**caractérisé par** un rapport d'aspect des orifices (22) traversants du substrat (20, 21, 30, 32, 34) est-compris entre 1 et 100, de préférence entre 5 et 50 ledit rapport d'aspect étant défini comme un rapport d'une longueur sur un diamètre d'un orifice traversant, en fonction du débit du flux de gaz entrant dans la source de plasma (10) ce dernier étant compris entre un ou plusieurs centimètres cubes par minutes standard (sccm) et quelques litres par minutes standards (slm) dans des conditions de référence de 20°C et 1013 10² Pa.

6. Dispositif (1) de génération selon la revendication 5, dans lequel le substrat (20, 21, 30, 32, 34) est formé dans l'un des matériaux suivants : un matériau diélectrique, un matériau conducteur, un matériau conducteur recouvert en cible (2) d'un matériau diélectrique, matériau diélectrique recouvert en surface d'un matériau conducteur, ou comprend une première partie formée dans un matériau diélectrique et une deuxième partie formée dans un matériau conducteur.

7. Dispositif (1) de génération selon l'une des revendications 5 ou 6, dans lequel le substrat (20, 21, 30, 32, 34) comprend une enveloppe (21) comprenant une première extrémité 21a) configurée pour recevoir le plasma et une deuxième extrémité (21b) opposée à la première extrémité (21a), les orifices (22) traversants étant répartis sur une surface de l'enveloppe (21) ou étant alignés entre la première et la deuxième extrémité (21a, 21b) de l'enveloppe (21).

8. Dispositif (1) de génération selon la revendication 7, dans lequel le substrat (20, 21, 30, 32, 34) comprend :
- un substrat (20) plan, s'étendant sensiblement perpendiculairement à une direction de propagation du jet de plasma primaire (3), les orifices (22) traversant étant formés dans une surface dudit substrat (20) plan,
- un substrat (21) de génération présentant une surface de forme cylindrique, comprenant une série d'orifices (22) traversants formés dans la surface de l'enveloppe (21) et configurés pour former autant de jets de plasma secondaire (4),
- une enveloppe (21) présentant une surface de forme cylindrique comprenant deux séries d'orifices (22) s'étendant dans un même plan le entre la première et la deuxième extrémité (21a, 21b) de l'enveloppe (21) et configurées pour former deux lignes de jets de plasma secondaire (4) s'étendant radialement depuis l'enveloppe (21),
- un tube présentant une surface sensiblement cylindrique de révolution dans laquelle sont formées une pluralité de lignes d'orifices (22) traversants configurées pour former une pluralité de lignes de jets de plasma secondaire (4),
- un tube interne (30) logé dans un tube externe (30), l'un parmi le tube interne (30) et le tube externe (32) étant sensiblement continu tandis que les orifices (22) traversants sont formés dans l'autre parmi le tube externe (32) et le tube interne (30), et/ou
- un goupillon (34), présentant une surface de forme sensiblement cylindrique de révolution et comprenant une pluralité d'orifices (22) traversants répartis sur sa surface.

9. Dispositif (1) de génération selon l'une des revendications 5 à 8, dans lequel le substrat (20) comprend plusieurs branches (38) connectées ensemble au niveau d'un noyau (36), ledit noyau (36) étant placé à proximité de la source de plasma (10).

10. Dispositif (1) de génération selon l'une des revendications 5 à 9, comprenant en outre un guide (18) formé dans un matériau diélectrique s'étendant entre la source de plasma (10) et le substrat (20, 21, 30, 32, 34), de manière à transporter le flux de gaz vers les orifices (22) traversants dudit substrat (20, 21, 30, 32, 34).

11. Dispositif (1) de génération selon la revendication 10, comprenant en outre un guide intermédiaire (19), disposé entre la source de plasma (10) et le guide (18) formé dans un matériau diélectrique, le guide (18) formé dans un matériau diélectrique étant connecté à une source de gaz plasmagène (F') et au substrat (20).

12. Dispositif (1) de génération selon la revendication 11, dans lequel le guide intermédiaire (19) est conducteur et connecté à une électrode (17) de la source de plasma (10) afin de former le jet de plasma primaire (3), ledit jet de plasma primaire étant ensuite transféré dans le guide (18) formé dans un matériau diélectrique.

## Patentansprüche

1. Verfahren (S) zur Erzeugung einer Vielzahl von Kaltplasmastrahlen bei Atmosphärendruck zum Behandeln eines Ziels (2), folgende Schritte umfassend:
- Herstellen (S1) eines primären Kaltplasmastrahls (3) bei Atmosphärendruck mithilfe einer Plasmaquelle (10) durch Erzeugung von elektrischen Entladungen in einem plasmagenen Gasfluss,
- Platzieren (S2) eines Substrates (20, 21, 30, 32, 34) in der Nähe des zu behandelnden Ziels (2), wobei das Substrat (20, 21, 30, 32, 34) mindestens zwei Durchgangsöffnungen (22) umfasst,
- Durchleiten (S3) des primären Plasmastrahls (3) durch die Durchgangsöffnungen (22) zum Erzeugen von mindestens zwei sekundären Kaltplasmastrahlen (4) bei Atmosphärendruck,
**gekennzeichnet durch** ein Seitenverhältnis der Durchgangsöffnungen (22) des Substrates (20, 21, 30, 32, 34), welches zwischen 1 und 100, vorzugsweise zwischen 5 und 50 beträgt, wobei das Seitenverhältnis als ein Verhältnis einer Länge zu einem Durchmesser einer Durchgangsöffnung definiert ist, gemäß dem Durchfluss des Gasflusses, welcher in die Plasmaquelle (10) eintritt, wobei letzterer zwischen einem und mehreren Standardkubikzentimetern pro Minute (sccm) und einigen Standardlitern pro Minute (slm) unter Referenzbedingungen von 20 °C und 1013 10² Pa beträgt.

2. Erzeugungsverfahren (S) nach Anspruch 1, ferner umfassend einen Schritt, in dessen Verlauf eine Führung (18), welche aus einem dielektrischen Material gebildet ist, zwischen der Plasmaquelle (10) und dem Substrat (20, 21, 30, 32, 34) in einer Weise platziert ist, dass der Gasfluss zu den Durchgangsöffnungen (22) des Substrates (20, 21, 30, 32, 34) transportiert wird.

3. Erzeugungsverfahren (S) nach Anspruch 2, wobei der Fluss des plasmagehenden Gases ein Edelgas umfasst, eine Mischung aus Edelgasen oder eine Mischung aus einem oder mehreren Edelgasen mit einer minderheitlichen Mischung, welche ein oder mehrere molekulare Gase umfasst.

4. Erzeugungsverfahren (S) nach Anspruch 3, wobei das zu behandelnde Ziel (2) eine hohle Struktur umfasst, wobei das Verfahren einen Teilschritt des Einführens des Substrates (34) in die hohle Struktur vor dem Schritt (S3) des Durchleitens des primären Plasmastrahls (3) durch die Durchgangsöffnungen (22) umfasst.

5. Vorrichtung (1) zur Erzeugung einer Vielzahl von Kaltplasmastrahlen bei Atmosphärendruck zum Behandeln eines Ziels (2), wobei die Vorrichtung Folgendes umfasst:
- eine Plasmaquelle (10), welche konfiguriert ist, um einen primären Kaltplasmastrahl (3) bei Atmosphärendruck durch Erzeugung von elektrischen Entladungen in einem plasmagenen Gasfluss herzustellen,
- ein Substrat (20, 21, 30, 32, 34), welches eine Vielzahl von Durchgangsöffnungen (22) umfasst, und
- Mittel, welche konfiguriert sind, um das Plasma durch die Durchgangsöffnungen (22) des Substrates (20, 21, 30, 32, 34) in einer Weise zu leiten, dass eine Vielzahl von sekundären Kaltplasmastrahlen (4) bei Atmosphärendruck erzeugt wird,
**gekennzeichnet durch** ein Seitenverhältnis der Durchgangsöffnungen (22) des Substrates (20, 21, 30, 32, 34), welches zwischen 1 und 100, vorzugsweise zwischen 5 und 50 beträgt, wobei das Seitenverhältnis als ein Verhältnis einer Länge zu einem Durchmesser einer Durchgangsöffnung definiert ist, gemäß dem Durchfluss des Gasflusses, welcher in die Plasmaquelle (10) eintritt, wobei letzterer zwischen einem und mehreren Standardkubikzentimetern pro Minute (sccm) und einigen Standardlitern pro Minute (slm) unter Referenzbedingungen von 20 °C und 1013 10² Pa beträgt.

6. Vorrichtung (1) zur Erzeugung nach Anspruch 5, wobei das Substrat (20, 21, 30, 32, 34) aus einem der folgenden Materialien gebildet ist: einem dielektrischen Material, einem leitfähigen Material, einem leitfähigen Material, welches am Ziel (2) mit einem dielektrischen Material bedeckt ist, einem dielektrischen Material, welches an der Oberfläche mit einem leitfähigen Material bedeckt ist, oder einen ersten Abschnitt umfasst, welcher aus einem dielektrischen Material gebildet ist, und einen zweiten Abschnitt, welcher aus einem leitfähigen Material gebildet ist.

7. Vorrichtung (1) zur Erzeugung nach einem der Ansprüche 5 oder 6, wobei das Substrat (20, 21, 30, 32, 34) eine Hülle (21) umfasst, welche ein erstes Ende (21a) umfasst, welches konfiguriert ist, um das Plasma aufzunehmen und ein zweites Ende (21b), welches dem ersten Ende (21a) entgegengesetzt ist, wobei die Durchgangsöffnungen (22) an einer Oberfläche der Hülle (21) verteilt oder zwischen dem ersten und dem zweiten Ende (21a, 21b) der Hülle (21) ausgefluchtet sind.

8. Vorrichtung (1) zur Erzeugung nach Anspruch 7, wobei das Substrat (20, 21, 30, 32, 34) Folgendes umfasst:
- ein ebenes Substrat (20), welches sich im Wesentlichen senkrecht zu einer Fortpflanzungsrichtung des primären Plasmastrahls (3) erstreckt, wobei Durchgangsöffnungen (22) in einer Oberfläche des ebenen Substrats (20) gebildet sind,
- ein Erzeugungssubstrat (21), welches eine Oberfläche mit einer zylindrischen Form aufweist, welche eine Reihe von Durchgangsöffnungen (22) umfasst, welche in der Oberfläche der Hülle (21) gebildet und konfiguriert sind, um ebenso viele sekundäre Plasmastrahlen (4) zu bilden,
- eine Hülle (21), welche eine Oberfläche mit einer zylindrischen Form aufweist, welche zwei Reihen von Öffnungen (22) umfasst, welche sich in einer gleichen Ebene zwischen dem ersten und dem zweiten Ende (21a, 21b) der Hülle (21) erstrecken und konfiguriert sind, um zwei Reihen von sekundären Plasmastrahlen (4) zu bilden, welche sich radial von der Hülle (21) weg erstrecken,
- eine Röhre, welche eine im Wesentlichen rotationszylinderförmige Fläche aufweist, in welcher eine Vielzahl von Reihen von Durchgangsöffnungen (22) gebildet sind, welche konfiguriert sind, um eine Vielzahl von Reihen von sekundären Plasmastrahlen (4) zu bilden,
- eine innere Röhre (30), welche in einer äußeren Röhre (30) untergebracht ist, wobei eine von der inneren Röhre (30) und der äußeren Röhre (32) im Wesentlichen durchgängig ist, während die Durchgangsöffnungen (22) in der anderen von der äußeren Röhre (32) und der inneren Röhre (30) gebildet sind, und/oder
- eine Flaschenbürste (34), welche eine im Wesentlichen rotationszylinderförmige Fläche aufweist und eine Vielzahl von auf ihrer Oberfläche verteilten Durchgangsöffnungen (22) umfasst.

9. Vorrichtung (1) zur Erzeugung nach einem der Ansprüche 5 bis 8, wobei das Substrat (20) mehrere miteinander auf Höhe eines Kerns (36) verbundene Zweige (38) umfasst, wobei der Kern (36) in der Nähe der Plasmaquelle (10) platziert ist.

10. Vorrichtung (1) zur Erzeugung nach einem der Ansprüche 5 bis 9, ferner umfassend eine Führung (18), welche aus einem dielektrischen Material gebildet ist, welche sich zwischen der Plasmaquelle (10) und dem Substrat (20, 21, 30, 32, 34) in einer Weise erstreckt, dass der Gasfluss zu den Durchgangsöffnungen (22) des Substrates (20, 21, 30, 32, 34) transportiert wird.

11. Vorrichtung (1) zur Erzeugung nach Anspruch 10, ferner umfassend eine Zwischenführung (19), welche zwischen der Plasmaquelle (10) und der aus einem dielektrischen Material gebildeten Führung (18) angeordnet ist, wobei die aus einem dielektrischen Material gebildete Führung (18) mit einer Quelle von plasmagenem Gas (F') und dem Substrat (20) verbunden ist.

12. Vorrichtung (1) zur Erzeugung nach Anspruch 11, wobei die Zwischenführung (19) leitfähig und mit einer Elektrode (17) der Plasmaquelle (10) verbunden ist, um den primären Plasmastrahl (3) zu bilden, wobei der primäre Plasmastrahl anschließend in die aus einem dielektrischen Material gebildete Führung (18) transferiert wird.

## Claims

1. A method (S) for generating a plurality of cold plasma jets at atmospheric pressure in order to treat a target (2), comprising the following steps:
- producing (S1) a primary cold plasma jet (3) at atmospheric pressure using a plasma source (10), by generating electrical discharges in a flow of plasmagene gas,
- placing (S2) a substrate (20, 21, 30, 32, 34) near the target (2) to be treated, said substrate (20, 21, 30, 32, 34) comprising at least two through holes (22),
- passing (S3) the primary plasma jet (3) through the through holes (22) such as to generate at least two secondary cold plasma jets (4) at atmospheric pressure, **characterized by** an aspect ratio of the through holes (22) of the substrate (20, 21, 30, 32, 34) comprised between 1 and 100, preferably between 5 and 50, said aspect ratio being defined as a ratio of a length to a diameter of a through hole, as a function of the gas flow entering the plasma source (10), the latter being comprised between one or several standard cubic centimeters per minute (sccm) and a few standard liters per minutes (slm), under reference conditions of 20 °C and 1013 10² Pa.

2. The generation method (S) according to claim 1, further comprising a step comprising a step wherein a guide (18) formed of a dielectric material placed between the plasma source (10) and the substrate (20, 21, 30, 32, 34), so as to transport the gas flow toward the through holes (22) of said substrate (20, 21, 30, 32, 34).

3. The generation method (S) according to claim 2, wherein the flow of plasmagene gas comprises a rare gas, a mixture of rare gases or a mixture of one or more rare gases with a minority mixture comprising one or more molecular gases.

4. The generation method (S) according to claim 3, wherein the target (2) to be treated comprises a hollow structure, the method comprising a sub-step of introducing the substrate (34) into said hollow structure prior to step (S3) consisting of passing the primary plasma jet (3) through the through holes (22).

5. A device (1) for generating a plurality of cold plasma jets at atmospheric pressure in order to treat a target (2), the device comprising:
- a plasma source (10), configured to produce a primary cold plasma jet (3) at atmospheric pressure, by generating electrical discharges in a flow of plasmagene gas,
- a substrate (20, 21, 30, 32, 34) comprising a plurality of through holes (22), and
- means configured to pass the plasma through the through holes (22) of the substrate (20, 21, 30, 32, 34) such as to generate a plurality of secondary cold plasma jets (4) at atmospheric pressure,
**characterized by** an aspect ratio of the through holes (22) of the substrate (20, 21, 30, 32, 34) being comprised between 1 and 100, preferably between 5 and 50, said aspect ratio being defined as a ratio of a length to a diameter of a through hole, as a function of the gas flow entering the plasma source (10), the latter being comprised between one or several standard cubic centimeters per minute (sccm) and a few standard liters per minutes (slm), under reference conditions of 20 °C and 1013 10² Pa.

6. The generation device (1) according to claim 5, wherein the substrate (20, 21, 30, 32, 34) is formed of one of the following materials: a dielectric material, a conductive material, a conductive material covered, at target (2), by a dielectric material, a dielectric material covered at its surface by a conductive material, or comprises a first portion formed of a dielectric material and a second portion formed of a conductive material.

7. The generation device (1) according to one of claims 5 or 6, wherein the substrate (20, 21, 30, 32, 34) comprises a sleeve (21) comprising a first end (21a) configured to receive the plasma and a second end (21b) opposite the first end (21a), the through holes (22) being distributed over a surface of the sleeve (21) or being aligned between the first and the second ends (21a, 21b) of the sleeve (21).

8. The generation device (1) according to claim 7, wherein the substrate (20, 21, 30, 32, 34) comprises:
- a flat substrate (20), extending substantially perpendicular to a propagation direction of the primary plasma jet (3), the through holes (22) being formed in a surface of said flat substrate (20),
- a generation substrate (21) having a surface with a cylindrical shape comprising a series of through holes (22) formed in the surface of the sleeve (21) and configured to form as many secondary plasma jets (4),
- a sleeve (21) having a surface with a cylindrical shape comprising two series of holes (22) extending in one and the same plane between the first and the second ends (21a, 21b) of the sleeve (21) and configured to form two lines of secondary plasma jets (4) extending radially from the sleeve (21),
- a tube having a substantially cylinder-of-revolution-shaped surface wherein a plurality of lines of through holes (22) are formed, which are configured to form a plurality of lines of secondary plasma jets (4),
- an internal tube (30) housed in an external tube (30), one of the internal tube (30) and the external tube (32) being substantially continuous while the through holes (22) are formed in the other of the external tube (32) and the internal tube (30), and/or
- a brush (34), having a surface with a substantially cylinder-of-revolution-shaped surface and comprising a plurality of through holes (22) distributed on its surface.

9. The generation device (1) according to one of claims 5 to 8, wherein the substrate (20) comprises several branches (38) connected together at a core (36), said core (36) being placed adjacent to the plasma source (10).

10. The generation device (1) according to one of claims 5 to 9, further comprising a guide (18) formed of a dielectric material extending between the plasma source (10) and the substrate (20, 21, 30, 32, 34), so as to transport the gas flow toward the through holes (22) of said substrate (20, 21, 30, 32, 34).

11. The generation device (1) according to claim 10, further comprising an intermediate guide (19) positioned between the plasma source (10) and the guide (18) formed of a dielectric material, the guide (18) formed of a dielectric material being connected to a plasmagene gas source (F') and to the substrate (20).

12. The generation device (1) according to claim 11, wherein the intermediate guide (19) is conductive and connected to an electrode (17) of the plasma source (10) so as to form the primary plasma jet (3), said primary plasma jet then being transferred in the guide (18) formed of a dielectric material.
